# EUROPEAN PATENT APPLICATION

(11) **EP 2 269 564 A2**
(43) Date of publication of application: **05.01.2011**
(21) Application number: 10005308.1
(22) Date of filing: 21.05.2010
(51) Int. Cl.: A61J 15/00, A61M 25/01

(54) **Gripping implement**

(30) Priority: 29.06.2009 JP 2009153765
(71) Applicant: Tyco Healthcare Group LP, Mansfield, MA 02048 (US)
(72) Inventor: Abe, Kazuhiro, Fukuroi Shizuoka 437-0004 (JP)
(74) Representative: Olsson, Carl H.S.

(57) **Abstract**

[Issue] To provide a gripping implement which does not become embedded inside a gastric fistula when a gastrostomy catheter is pushed into the body, and which makes it possible not to have to touch the patient's body or the gastrostomy catheter directly with the hands when a specific operation is carried out with the gastrostomy catheter.

[Means of Resolution] A gripping implement 10, which is used for carrying out specific operations with a gastrostomy catheter 20 having a tubular part 22 which is placed in a gastric fistula and an external holding part 21 which is linked to the tubular part 22 and is placed at the abdominal wall surface of the gastric fistula, consists of an engaging part 11, a gripping part 12 and a linking part 13. The linking part 13 links the engaging part 11 and the gripping part 12 and is designed to position the gripping part 12 in a position away from the direction of extension a fluid flow passage formed inside the gastrostomy catheter 20 when the engaging part 11 has engaged with the external holding part 21. Furthermore, the engaging part 11 comprises a support part 11a consisting of a pair of support pieces, and a pair of gripping pieces 11b.

## Description

### [Technical Field]

The present invention relates to a gripping implement which is used when a specific operation is carried out with a gastrostomy catheter which is employed in order to supply fluids such as nutrients and food in fluid form into a patient's gastrointestinal tract.

### [Prior Art]

Fluids such as nutrients or food in fluid form are conventionally supplied to people having a reduced capacity for ingesting food orally by themselves due to advanced age or illness (referred to hereinafter as "patient(s)") using a gastrostomy catheter. The gastrostomy catheter generally comprises a tubular part which passes through a gastric fistula for ingestion which is formed in the patient's abdomen, an external holding part which is attached to the base end of the tubular part and placed on the surface of the skin at the abdomen, and an internal holding part which is attached to the tip end of the tubular part and is placed inside the gastrointestinal tract, in the stomach, for instance. This kind of gastrostomy catheter is fitted in a gastric fistula formed in the patient's abdomen, and the wall of the gastrointestinal tract and the abdomen wall are fixed, after which the catheter is connected to a fluid supply tube and fluid which is delivered via this fluid supply tube is supplied to the gastrointestinal tract.

If the fluid supply tube is pressed strongly into this kind of gastrostomy catheter when the fluid supply tube is connected to the gastrostomy catheter, there is a risk of the external holding part of the gastrostomy catheter becoming embedded in the gastric fistula inside the body. In order to prevent the external fixing part from becoming embedded in the gastric fistula inside the body, the fluid supply tube needs to be connected to the gastrostomy catheter without the fluid supply tube strongly pressing the gastrostomy catheter. For this reason, there is a component in the gastrostomy catheter which is provided so that the fluid supply tube is made to engage with the external holding part of the gastrostomy catheter from a direction perpendicular to the longitudinal direction of the gastrostomy catheter (see Patent Document 1, for example).

With this catheter for establishing a gastric fistula (gastrostomy catheter), a flange is formed at the top of an adapter (external holding part), and legs which can engage with the flange of the catheter for establishing a gastric fistula are provided at the tip end of a feeding tube assembly (fluid supply tube). The feeding tube assembly is designed to be connected in a state in which it is perpendicular to the longitudinal direction of the catheter for establishing a gastric fistula, and the tip-end opening thereof is formed to be perpendicular to the feeding tube of the feeding tube assembly. A space which is the thickness of the flange from the tip-end opening is then provided in order to provide for the legs. Moreover, the base end of the legs is coupled to the tip end of the feeding tube assembly.

### [Prior Art Document]

### [Patent Document]

[Patent Document 1] Published Japanese Translation of a PCT Application 2008-504896

### [Summary of the Invention]

Leaked fluid often clings to the gastrostomy catheter and the region around the gastric fistula in the patient's body, leading to soiling. For this reason, it is sometimes undesirable to touch the patient's body and the gastrostomy catheter directly with the hands. However, operations with the conventional gastrostomy catheter described above are carried out by holding the external holding part directly with the hands. Furthermore, the fluid passage of the gastrostomy catheter and the fluid passage of the fluid supply tube which is connected to the gastrostomy catheter are at right-angles, and therefore there are problems in that the flow of fluid from the fluid supply tube to the gastrostomy catheter is not smooth, and there are further problems in that the fluid is likely to leak at the connection between the gastrostomy catheter and the fluid supply tube.

The present invention has been devised in order to resolve the problems mentioned above, and it aims to provide a gripping implement which does not become embedded inside a gastric fistula when a gastrostomy catheter is pushed into the body, and which makes it possible not to have to touch the patient's body or the gastrostomy catheter directly with the hands when a specific operation is carried out with the gastrostomy catheter.

In order to achieve the aim described above, structural features of the gripping implement according to the present invention lie in the fact that it is a gripping implement which is used for carrying out specific operations with a gastrostomy catheter which is provided with a tubular part that is placed in a gastric fistula formed in a patient's abdominal wall and the wall of the gastrointestinal tract, and an external holding part which is linked to the tubular part and is placed at the abdominal wall surface of the gastric fistula, said gripping implement having: an engaging part which can engage with the external holding part; a gripping part which is disposed at a specific distance from the engaging part; and a linking part which links the engaging part and the gripping part and is designed to position the gripping part in a position away from the direction of extension of a fluid flow passage formed inside the gastrostomy catheter when the engaging part has been engaged with the external holding part.

The gripping implement according to the present invention has the engaging part which can engage with the external holding part, a gripping part which is disposed at a specific distance from the engaging part, and a linking part which links the engaging part and the gripping part, and the linking part positions the gripping part in a position away from the direction of extension of the fluid passage formed in the gastrostomy catheter when the engaging part has been engaged with the external holding part. Accordingly, when a specific operation is carried out with the gastrostomy catheter while the gripping part is held by the hand in a state in which the engaging part has been engaged with the external holding part, the operation can be carried out without the patient's body or the gastrostomy catheter being touched directly with the hands.

Furthermore, even if the specific operation involves pressing the external holding part towards the patient's body, the gripping part of the gripping implement in which the engaging part has engaged with the external holding part is manipulated so as not to press the external holding part into the patient's body, whereby the external holding part can be prevented from becoming embedded in the gastric fistula. In the present invention, the gripping part is positioned away from the direction of extension of the fluid passage of the gastrostomy catheter, for example the gripping part is positioned at a place which is not above the external holding part when the external holding part is positioned above the gastrostomy catheter, and therefore the gripping part does not obstruct manipulation of the external holding part. Note that the specific operation according to the present invention is an operation to connect another tube or similar to the gastrostomy catheter, an operation to open or close the opening of the gastrostomy catheter with a cover such as a cap, or an operation to clean the region of the patient's body around the gastrostomy catheter, or another operation.

Further structural features of the gripping implement according to the present invention lie in the fact that the engaging part has a pair of support pieces which lie either side of the tubular part and support the surface of the external holding part on the tubular part side. This means that the surface of the external holding part on the tubular part side can be supported by the pair of support pieces, and therefore the external holding part can be prevented from being pressed into the patient's body. As a result, the external holding part can be reliably prevented from becoming embedded in the gastric fistula.

Further structural features of the gripping implement according to the present invention lie in the fact that the engaging part which has the pair of support pieces comprises a substantially plate-like section which extends beyond the surface of the external holding part on the tubular part side. This means that the surface of the engaging part which is in contact with the abdominal wall surface is increased, and therefore even if the force whereby the engaging part is pressed against the abdominal wall increases, this force is dispersed, so the patient is not subjected to pain and the external holding part can be stably supported. Moreover, in this case, the section of the engaging part which comes into contact with the external holding part may comprise a rigid resin layer or a metal layer such as stainless steel, and the section which comes into contact with the abdominal wall may comprise a layer made of a soft material such as silicone.

Further structural features of the gripping implement according to the present invention lie in the fact that the external holding part comprises an insertion opening having a connection orifice, and protrusions which extend from both sides of the insertion opening in a direction orthogonal to the fluid passage; and the engaging part is provided with a pair of gripping pieces which support the surfaces of the protrusions on the opposite side to the tubular part, in a state in which the side parts of the insertion opening are gripped. This means that both surfaces of both protrusions lie between the pair of support pieces and the pair of gripping pieces, while the side parts of the external holding part can be gripped by the pair of gripping pieces, and therefore the engaging part can be securely engaged with the external holding part.

Further structural features of the gripping implement according to the present invention lie in the fact that the linking part extends from the engaging part at an inclination or curved with respect to the direction of extension of the fluid passage which is formed inside the gastrostomy catheter. This means that the gripping part is positioned in a place which is sufficiently remote from the direction of extension of the fluid passage formed in the gastrostomy catheter that the external holding part is simple to handle.

Further structural features of the gripping implement according to the present invention lie in the fact that the specific operation is an operation to connect a fluid supply tube for supplying a fluid into the gastrointestinal tract of a patient to the external holding part of the gastrostomy catheter. This means that the gastrostomy catheter is not pressed into the patient's body when the fluid supply tube is pressed into the gastrostomy catheter to connect it thereto.

### [Brief Description of the Figures]

[Figure 1] is an oblique view showing the gripping implement according to a mode of embodiment of the present invention;
[Figure 2] is a side view showing a state in which a fluid supply tube is to be connected to the gastrostomy catheter which has been assembled with a connecting member and the gripping implement;
[Figure 3] shows the gastrostomy catheter, where (a) is a plan view and (b) is a front view;
[Figure 4] is a bottom view of the connecting member;
[Figure 5] is a side view showing a state in which the fluid supply tube has been connected to the gastrostomy catheter which has been assembled with the connecting member and the gripping implement;
[Figure 6] is a side view showing a state in which the gripping implement has been removed from the state shown in Figure 5; and
[Figure 7] is a front view showing a state in which a fluid supply tube has been connected to a gastrostomy catheter using the gripping implement, in accordance with a variant example.

### [Mode of Embodiment of the Invention]

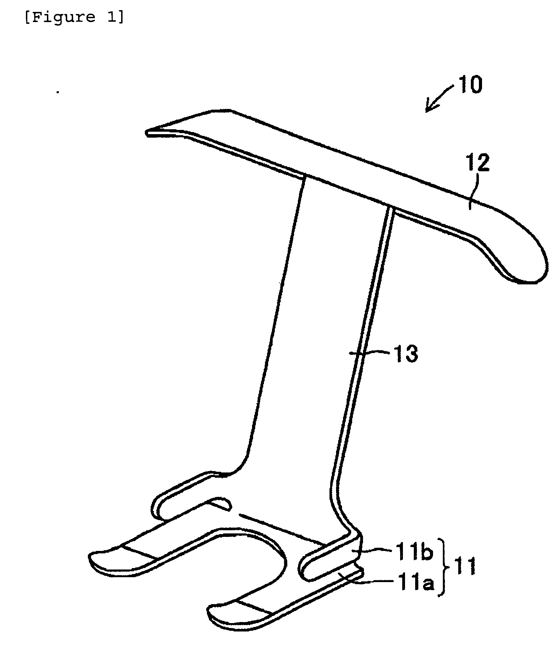

A mode of embodiment of the present invention will be described below with the aid of the figures. Figure 1 shows a gripping implement 10 according to this mode of embodiment. The gripping implement 10 is formed into the shape shown in Figure 1 by working a stainless steel sheet, and it is provided with an engaging part 11, a gripping part 12, and a linking part 13 which links the engaging part 11 and gripping part 12. The engaging part 11 side shall be referred to hereinafter as the lower side/bottom/below and the gripping part 12 side as the upper side/top/above, and the surface shown in Figure 1 shall be referred to as the front. The engaging part 11 comprises: a support part 11a which is substantially U-shaped when seen from the front, and which is formed at right-angles to the linking part 13 and extends in a horizontal direction from the lower end of the linking part 13 towards the front in the figure; and a pair of gripping pieces 11b which are provided parallel to the support part 11a and spaced apart from both sides of the support part 11a; these pieces lie at right-angles to the linking part 13 and extend from both the left and right sides of the section at the lower end of the linking part 13.

Moreover, the two side portions of the substantially U-shaped support part 11a constitute the pair of support pieces according to the present invention, and the tip ends of this pair of support pieces are slightly curved upwards. Furthermore, the pair of gripping pieces 11b consist of elongate plate-like pieces which extend forwards from the two sides of the section at the lower end of the linking part 13, with the width direction being the vertical direction and the thickness direction being the lateral direction, and the length thereof in the front-to-rear direction is shorter than the length of the support part 11a in the front-to-rear direction. A gap is then provided between the two sides of the support part 11a and the gripping pieces 11b lying opposite. Furthermore, the length of the linking part 13 in the vertical direction is set at 50 - 300 mm, preferably 60 - 100 mm.

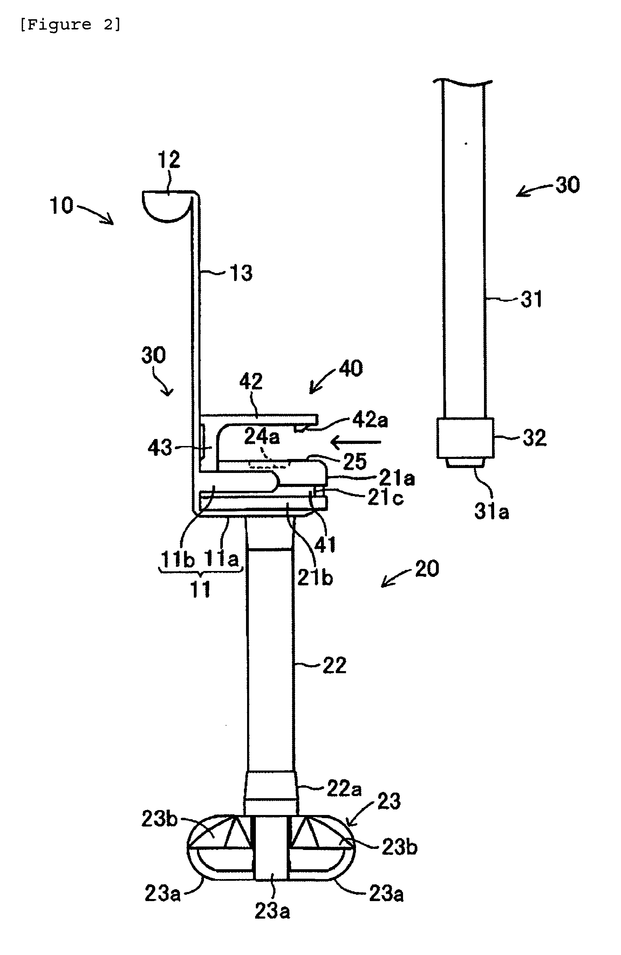

The gripping part 12 lies at right-angles to the linking part 13, protruding from the upper end of the linking part 13 horizontally towards the rear, and it is formed on the opposite side to the engaging part 11, with the linking part 13 in between when seen as a planar view. The gripping part 12 consists of a plate-like piece which is long in the transverse direction and extends as a protrusion to both the left and right of the linking part 13. Furthermore, the length of the gripping part 12 in the front-to-rear direction is set to be short, and the two side sections to the left and right of the gripping part 12 are curved downwards in order to make it easier to grip with the hand. The gripping implement 10 which is configured in this manner is assembled for use with a gastrostomy catheter 20 when a fluid supply tube 30 is connected to the gastrostomy catheter 20, as shown in Figure 2, for example. In such cases, a connecting member 40 is also assembled with the gastrostomy catheter 20.

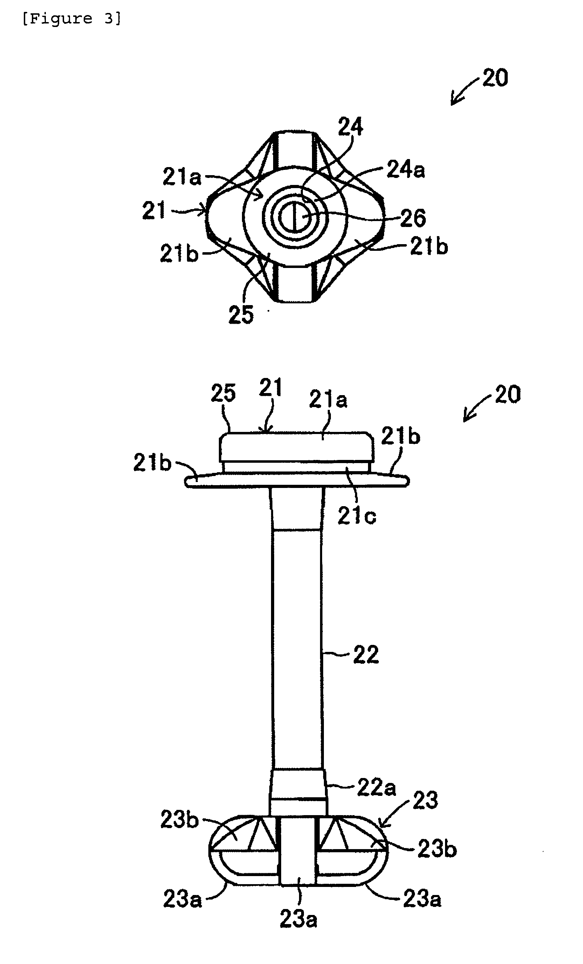

As shown in Figure 3, the gastrostomy catheter 20 comprises an external holding part 21, a tubular part 22 which is joined at the centre on the lower surface of the external holding part 21, and an internal holding part 23 which is joined to the tip end of the tubular part 22. The external holding part 21 comprises an insertion opening 21a which is formed as a fairly thick annular shape, and a pair of protrusions 21b which project outwards from both sides at the lower end on the outer periphery of the insertion opening 21a. The protrusions 21b have the function of preventing the gastrostomy catheter 20 from becoming embedded inside the patient's body. Furthermore, a fitting groove 21c for the attachment of the connecting member 40 is formed in the peripheral direction on the upper side of the protrusions 21b, at the outer peripheral surface of the insertion opening 21a.

An insertion hole 24 running vertically is then formed at the centre of the insertion opening 21a, and the diameter of an upper opening 24a of the insertion hole 24 becomes steadily greater from the bottom towards the top. Furthermore, the diameter of the top part on the outer periphery of the insertion opening 21a becomes steadily smaller from the bottom towards the top, and by means of this the centre of the top part on the outer periphery of the insertion opening 21a is formed as a raised curved face. By forming the tapered upper opening 24a and the curved face at the top part on the outer periphery, an annular raised part 25 is formed at the upper face of the insertion opening 21a. A check valve 26 which has a slit formed in the centre is then provided on the inner peripheral surface of the insertion hole 24.

The tubular part 22 consists of an elongate cylindrical body inside which is formed a fluid passage (not depicted) for the passage of fluids such as nutrients and food in fluid form, and the upper end of the fluid passage communicates with the insertion hole 24 of the external holding part 21. The internal holding part 23 is connected to the tubular part 22 by way of a connector 22a which is fixed to the lower end of the tubular part 22. The internal holding part 23 comprises four strip-shaped linking parts 23a which extend in four directions from the edge of the opening at the lower end of the connector 22a, and four linking film parts 23b which are provided between the upper parts of each of the linking parts 23a, and the tip ends of the linking parts 23a are linked to one another.

The fluid supply tube 30 comprises a tube main body 31 and a cylindrical engaging part 32 which is attached towards the lower end of the tube main body 31. The tube main body 31 consists of a soft tube having a fluid passage which communicates with the fluid passage of the tubular part 22, and it is substantially the same thickness as the tubular part 22. The tip end 31a of the tubular main body 31 enters the engaging part 32 by contracting under pressure in the axial direction.

Furthermore, the tip end 31a of the tube main body 31 can be fitted in a liquid-tight state with the upper section of the external holding part 21 including the upper opening 24a of the insertion hole 24, and by means of this fluid is able to flow to the fluid passage of the gastrostomy catheter 20 from the fluid passage in the tube main body 31. Moreover, the slit in the check valve 26 opens under pressure from the force of the fluid flowing from the tube main body 31 to the gastrostomy catheter 20. The engaging part 32 consists of a cylindrical body made of a rigid resin material, and it is fixed to the outer periphery at the lower end of the tube main body 31 with the tip end 31a of the tube main body 31 able to be received therein.

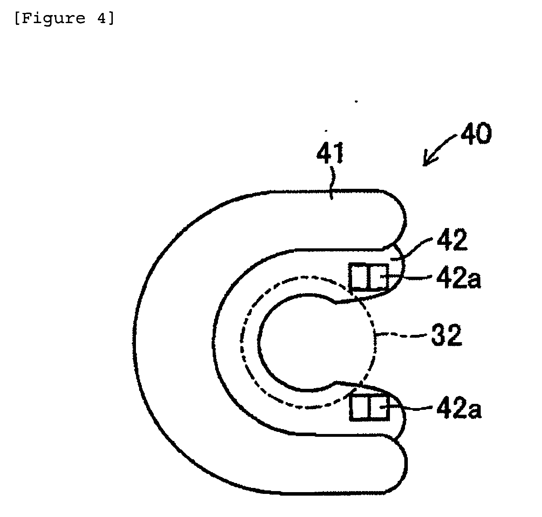

The connecting member 40 comprises a fitting part 41 which fits into the fitting groove 21c of the external holding part 21, a guide part 42 which is disposed parallel to and spaced apart from the fitting part 41, and a linking part 43 which links the fitting part 41 and the guide part 42. Figure 4 shows the connecting member 40 seen from the bottom, and the fitting part 41 consists of a substantially U-shaped plate-like piece. The inside section on the substantially U-shaped inner peripheral edge of the fitting part 41 fits into the fitting groove 21c, whereby the connecting member 40 is rotatably assembled with the external holding part 21 of the gastrostomy catheter 20. The guide part 42 consists of a substantially C-shaped plate when seen from above (not depicted), and it is arranged parallel to the fitting part 41 and spaced apart from the fitting part 41.

The space between the fitting part 41 and the guide part 42 is set so that the space between the upper surface of the raised part 25 of the external holding part 21 and the lower surface of the guide part 42 is substantially equal to the length of the engaging part 32 in the axial direction when the fitting part 41 has been fitted into the fitting groove 21c. Furthermore, the inner peripheral edge of the guide part 42 is formed in such a way that that the width between opposing sections on the open side (the right-hand section in Figure 4) becomes steadily narrower moving from the open side to the inside, and the inside thereof is formed to become substantially circular (C-shaped). The narrowest width between opposing sections on the open side of the guide part 42 is set to be greater than the outer diameter of the tube main body 31, and smaller than the outer diameter of the engaging part 32. Respective projections 42a are then formed at both ends on the lower surface of the guide part 42.

When the lower surfaces of these projections 42a are seen from the side, the portions lying on the open side of the guide part 42 are formed as tapers which are oriented steadily downwards moving from the open side to the inside of the guide part 42, and the portions lying on the inside of the guide part 42 are formed to be horizontal. Furthermore, the spacing of the pair of projections 42a is somewhat narrower than the outer diameter of the engaging part 32. As shown by the two-dot chain line in Figure 4, when the engaging part 32 has engaged with the guide part 42, the pair of projections 42a are in contact with the outer peripheral surface of the engaging part 32, and the engaging part 32 is prevented from being removed from the guide part 42. The linking part 43 links the central part of the top face of the fitting part 41 and the central part of the bottom face of the guide part 42, and it consists of a plate-like linking piece which has an arcuate shape in cross section (not depicted).

The connecting member 40 is assembled with the external holding part 21 of the gastrostomy catheter 20 by fitting the fitting part 41 into the fitting groove 21c, and the gripping implement 10 can be assembled with the external holding part 21 which is in this state. That is to say, the support part 11a of the gripping implement 10 is formed with a shape which allows the tubular part 22 to enter the inner portion thereof so that the bottom surface of the external holding part 21 is supported, and when the bottom surface of the external holding part 21 has been supported, as shown in Figure 2, the tip end of the support part 11a is positioned in the region of the front end of the external holding part 21, when seen from the side. Furthermore, the space between the two side parts of the support part 11a and the pair of gripping pieces 11b lying opposite is set to be a length which allows the protrusions 21b to be held in between. The space between the pair of gripping pieces 11b is set to be a size which allows the insertion opening 21a to be held from both sides.

With this configuration, when an operation is carried out with the gastrostomy catheter 20, for example an operation to connect the fluid supply tube 30 to the gastrostomy catheter 20 which has been fitted to the gastric fistula (not depicted) in the patient, the fitting part 41 is first of all fitted into the fitting groove 21c of the external fitting part 41 to assemble the connecting member 40 with the gastrostomy catheter 20. In this case, the two ends on the open side of the fitting part 41 are pushed into the two sides of the fitting groove 21c, and the connecting member 40 slides laterally with respect to the gastrostomy catheter 20. By means of this, the inside section on the inner peripheral edge of the fitting part 41 fits into the semicircular portion of the fitting groove 21c, and the connecting member 40 is assembled in a state in which it can rotate in the axial direction with respect to the gastrostomy catheter 20.

Next, the tubular part 22 is inserted into the support part 11a, and the support part 11a is made to slide from a transverse direction at the lower surface of the external holding part 21 so that the gripping implement 10 is assembled with the gastrostomy catheter 20, as shown in Figure 2. At this point, the two protrusions 21b are held between the support part 11a and the pair of gripping pieces 11b, and the insertion opening 21a is held from both sides by the pair of gripping pieces 11b, whereby the gripping implement 10 is securely assembled with the gastrostomy catheter 20 without any unsteadiness. The engaging part 32 is then positioned in the region between the open section of the guide part 42 of the connecting member 40 and the raised part 25 of the gastrostomy catheter 20, and the fluid supply tube 30 is brought closer to the gastrostomy catheter 20.

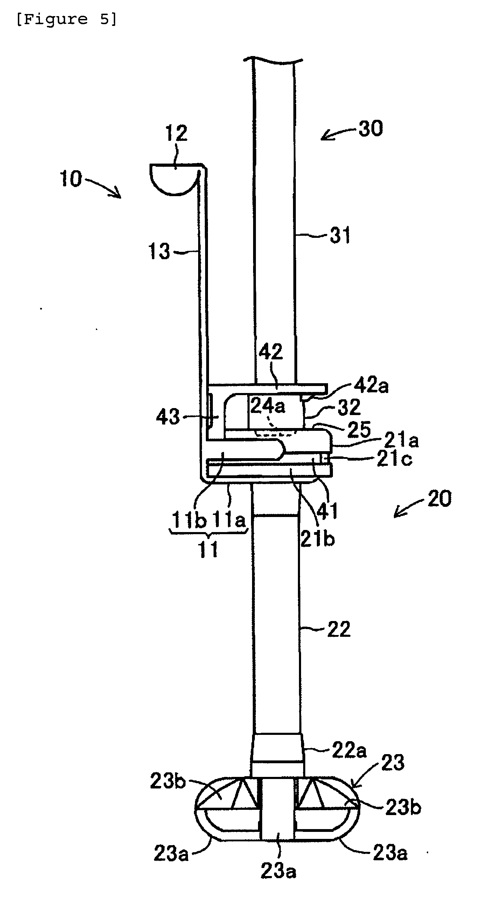

Then, as shown by the arrow in Figure 2, the fluid supply tube 30 is moved towards the gastrostomy catheter 20 so that the engaging part 32 is pushed between the raised part 25 and the guide part 42. At this time, the tip end 31a of the tube main body 31 is pressed by the raised part 25 and is received inside the engaging part 32, and the engaging part 32 enters between the raised part 25 and the guide part 42, while the left and right sides at the upper end of the engaging part 32 (the front and rear sides in the state shown in Figure 2) push the tapered surfaces of the projections 42a upwards. At this point, the open section of the guide part 42 is bent upwards. Then, when the upper ends on both sides of the engaging part 32 have gone past the projections 42a, the tip end 31a of the tube main body 31 protrudes from the engaging part 32 so as to fit into the top section of the insertion hole 24 of the external holding part 21, and the state shown in Figure 5 is achieved.

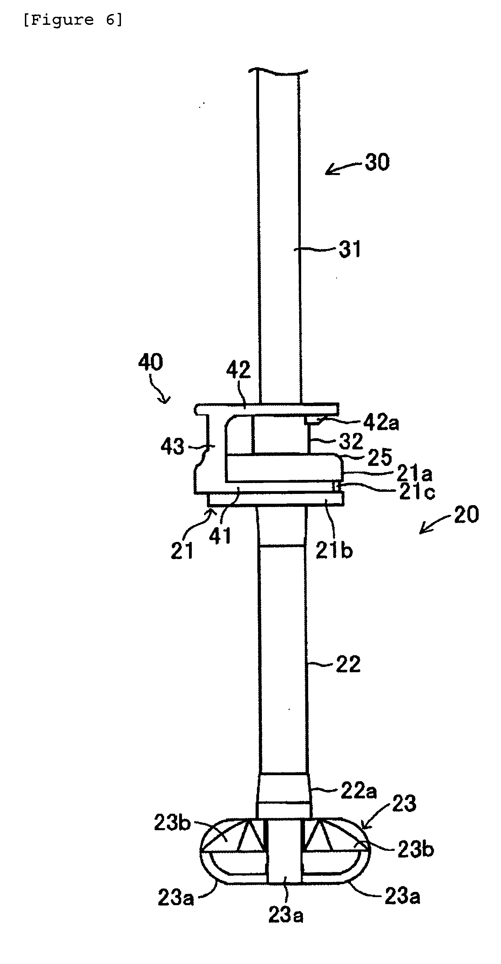

In this process, the tip end 31a of the tube main body 31 is in liquid-tight contact with the upper opening 24a. Furthermore, the guide part 42 returns to its original state and the projections 42a engage at the outer peripheral surface of the engaging part 32, as shown in Figure 4. By means of this, the engaging part 32 is prevented from being removed from the gastrostomy catheter 20. The operation at this time is carried out by holding the gripping part 12 of the gripping implement 10 with one hand, and holding the engaging part 32 with the other hand. Consequently, the series of operations can be carried out with virtually no force being applied to the gastrostomy catheter 20, and with no contact being made with the patient's body. Then, when the fluid supply tube 30 is connected to the gastrostomy catheter 20, the gripping implement 10 is removed from the gastrostomy catheter 20, and the state shown in Figure 6 is achieved. In this case, the gripping implement 10 can be easily removed from the gastrostomy catheter 20 by pulling the implement backwards.

In this state, fluid enters the tube main body 31 from the base end opening of the tube main body 31. Note that a supply apparatus housing the fluid has already been connected to the base end opening of the tube main body 31, and the fluid is supplied from the supply apparatus. As a result, the fluid is supplied from the fluid passage of the tube main body 31, through the insertion hole 24 and fluid passage of the tubular part 22, and into the patient's gastrointestinal tract, for example into the stomach. Furthermore, if fluid or soiling adheres to the external holding part 21 of the gastrostomy catheter 20, the fluid or soiling is wiped off using a specific cloth or paper.

In this case, the connecting member 40 is turned to allow the whole surface of the external holding member 31 to be wiped. At this point, the gripping implement 10 may be assembled with the gastrostomy catheter 20, if required. In addition, when the fluid supply tube 30 is removed from the gastrostomy catheter 10, the operation is carried out by assembling the gripping implement 10 with the external holding part 21 of the gastrostomy catheter 22 and holding the gripping part 12 of the gripping implement 10 and the engaging part 32 with the hands.

As described above, the gripping implement 10 according to this mode of embodiment has the engaging part 11 which can engage with the external holding part 21 of the gastrostomy catheter 20, the gripping part 12 which is disposed at a specific distance from the engaging part 11, and the linking part 13 which links the engaging part 11 and the gripping part 12. Then, when the engaging part 11 has been engaged with the external holding part 21, the gripping part 12 is positioned on the opposite side to the external holding part 21 with the linking part 13 in between. Accordingly, when a specific operation is carried out with the gastrostomy catheter 20 by holding the gripping part 12 in the hand while the gripping implement 10 is assembled with the gastrostomy catheter 20, this operation can be carried out without directly touching the patient's body or the gastrostomy catheter 20 with the hands.

Furthermore, the gripping part 12 is positioned at a place which is not above the external holding part 21, and therefore the gripping part 12 does not obstruct handling of the external holding part 21. In addition, the engaging part 11 consists of the engaging part 11a which comprises the pair of support pieces for supporting the lower surface of the external holding part 21 with the tubular part 22 in between, and the pair of gripping pieces 11b for gripping the sides of the insertion opening 21a of the external holding part 21. Furthermore, the support part 11a and the pair of gripping pieces 11b are disposed so as to be able to hold the two protrusions 21b in between. Accordingly, the engaging part 11 can be easily attached to and detached from the external holding part 21, and also the engaging part 11 can be securely engaged with the external holding part 21.

Furthermore, the engaging part 32 provided at the tip end of the tube main body 31 of the fluid supply tube 30 is pushed in to engage between the external holding part 21 and the guide part 42, whereby the tip end 31a of the tube main body 31 is placed in communication with the upper opening 24a in the external holding part 21. Accordingly, the fluid supply tube 30 can be connected to the gastrostomy catheter 20 by pushing the engaging part 32 between the external holding part 21 and the guide part 42 from the side, and there is no need to push the fluid supply tube 30 from above the gastrostomy catheter 20.

Furthermore, the tip end 31a of the tube main body 31 is made of a soft material, so when the tip end 31a is pressed, the tip end 31a contracts and is received inside the engaging part 32, and therefore the operation to make the engaging part 32 engage between the external holding part 21 and the guide part 42 is simplified. In addition, the respective projections 42a are formed at the lower surface at both ends of the guide part 42 so that when the engaging part 32 has engaged between the external holding part 21 and the guide part 42, the projections 42a are designed to engage on the side of the engaging part 32, and therefore the engagement between the engaging part 32 and the guide part 42 is more secure.

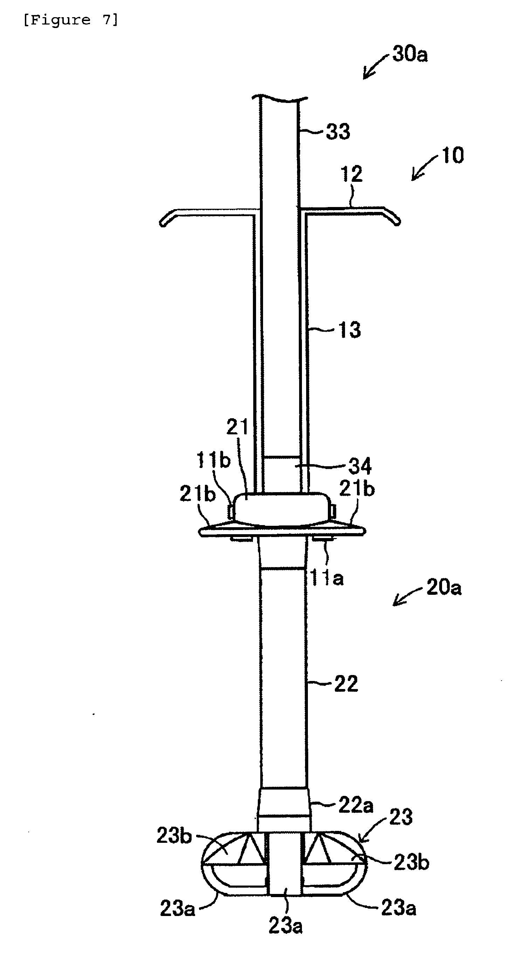

Figure 7 shows a state in which a fluid supply tube 30a has been connected to a gastrostomy catheter 20a using only the gripping implement 10, and without the use of the connecting member 40. This gastrostomy catheter 20a is not provided with the fitting groove 21c, but the other structural components of the gastrostomy catheter 20a are the same as those of the gastrostomy catheter 20. Accordingly, the same components bear the same reference symbols and they will not be described here. Furthermore, the fluid supply tube 30a has a configuration in which a cylindrical connector 34 which can engage with the insertion hole 24 provided in the external holding part 21 of the gastrostomy catheter 20a is connected to the tip end of the tube main body 33.

When the fluid supply tube 30a which is configured in this manner is connected to the gastrostomy catheter 20a, the gripping implement 10 is assembled with the gastrostomy catheter 20a using the method described above, and without the use of the connecting member 40. The connector 34 of the fluid supply tube 30a is then pushed into the insertion hole 24 in the external holding part 21, and the fluid supply tube 30a is placed in communication with the gastrostomy catheter 20a. When the connector 34 of the fluid supply tube 30a is pushed into the insertion hole 24 in the external holding part 21, the gripping part 12 of the gripping implement 10 is lifted up to prevent the gastrostomy catheter 20a from becoming embedded in the gastric fistula. The other operational effects of this variant example are the same as those of the mode of embodiment described above.

The gripping implement according to the present invention is not limited to the mode of embodiment described above, and suitable modifications may be made within the technical scope of the present invention. For example, in the mode of embodiment described above, the engaging part 11 comprises the support part 11a and the pair of gripping pieces 11b, but the engaging part according to the present invention is not limited by this, and it may comprise only the support part 11a. Even if the engaging part 11 comprises only the support part 11a, the gastrostomy catheter 20a can still be prevented from being pressed and embedded in the gastric fistula. Furthermore, instead of the support part 11a, the support part may, for example, consist of a member in which a cutaway part is provided in a fairly large square sheet, the cutaway part extending from the middle of one side towards the centre of the sheet and allowing the tubular part 22 to pass through to the inside.

This means that the surface of the engaging part which is in contact with the abdominal wall surface is increased, and therefore even if the force whereby the engaging part is pressed against the abdominal wall increases, this force is dispersed, so the patient is not subjected to pain and the external holding part 21 can be stably supported. Furthermore, the section of the engaging part which comes into contact with the external holding part 21 may comprise a rigid resin and the section which comes into contact with the abdominal wall may comprise a soft material, so that the engaging part has a two-layer structure. This means that it is possible to prevent the abdominal wall from becoming irritated, while also allowing secure engagement of the engaging part with the external holding part 21. In addition, any kind of member may be used for the engaging part, provided that it can be detachably engaged with the external holding part 21.

Furthermore, as shown in Figures 2 and 5, the linking part 13 lies at right-angles to the support part 11a and gripping part 12 in the mode of embodiment described above, but the linking part 13 may be inclined or curve with respect to the direction of extension of the gastrostomy catheter 20, so as to extend to the left and upwards from the engaging part 11 in Figures 2 and 5. This means that the gripping part 12 is positioned at a place sufficiently remote from the area above the gastrostomy catheter 20, and therefore the external holding part 21 can be handled more easily. Furthermore, for the gastrostomy catheter, use may be made of a catheter which does not have an internal holding part, or a catheter in which the tubular part is longer, and the internal holding part is joined further towards the base end than the tip end. In addition, a cap which can fit into the upper opening 24a may be provided on the external holding part 21 of the gastrostomy catheter 20, and when the fluid supply tube 30 is not connected to the gastrostomy catheter 20, the upper opening 24a may be closed off by the cap. The gripping implement 10 may be used when the cap is handled in this case.

### [Key to Symbols]

10...gripping implement; 11...engaging part; 11a...support part; 11b...gripping piece; 12...gripping part; 13...linking part; 20, 20a...gastrostomy catheter; 21...external holding part; 21a...insertion opening; 21b...protrusion; 22...tubular part; 30, 30a...fluid supply tube.

## Claims

1. Gripping implement which is used for carrying out specific operations with a gastrostomy catheter which is provided with a tubular part that is placed in a gastric fistula formed in a patient's abdominal wall and the wall of the gastrointestinal tract, and an external holding part which is linked to the tubular part and is placed at the abdominal wall surface of the gastric fistula,
said gripping implement being **characterized in that** it has: an engaging part which can engage with the external holding part; a gripping part which is disposed at a specific distance from the engaging part; and a linking part which links the engaging part and the gripping part and is designed to position the gripping part in a position away from the direction of extension of a fluid flow passage formed inside the gastrostomy catheter when the engaging part has been engaged with the external holding part.

2. Gripping implement according to Claim 1, in which the engaging part has a pair of support pieces which lie either side of the tubular part and support the surface of the external holding part on the tubular part side.

3. Gripping implement according to Claim 2, in which the engaging part which has the pair of support pieces comprises a substantially plate-like section which extends beyond the surface of the external holding part on the tubular part side.

4. Gripping implement according to Claim 2 or 3, in which the external holding part comprises an insertion opening having a connection orifice, and protrusions which extend from both sides of the insertion opening in a direction orthogonal to the fluid passage; and the engaging part is provided with a pair of gripping pieces which support the surface of the protrusions on the opposite side to the tubular part, in a state in which the side parts of the insertion opening are gripped.

5. Gripping implement according to any one of Claims 1 to 4, in which the linking part extends from the engaging part at an inclination or curved with respect to the direction of extension of the fluid passage which is formed inside the gastrostomy catheter.

6. Gripping implement according to any one of Claims 1 to 5, in which the specific operation mentioned above is an operation to connect a fluid supply tube for supplying a fluid into the gastrointestinal tract of a patient to the external holding part of the gastrostomy catheter.
